# EUROPEAN PATENT APPLICATION

(11) **EP 4 835 459 A1**
(43) Date of publication of application: **04.11.2026**
(21) Application number: 24912343.1
(22) Date of filing: 10.12.2024
(51) Int. Cl.: G01N 25/18, G01N 27/12

(54) **GAS ANALYSIS DEVICE, GAS ANALYSIS METHOD, AND PROGRAM FOR GAS ANALYSIS DEVICE**

(30) Priority: 28.12.2023 JP 2023222509
(71) Applicant: HORIBA, Ltd., Kyoto-shi Kyoto 601-8510 (JP)
(72) Inventor: MIZUTANI, Naoto, Kyoto-shi, Kyoto 601-8510 (JP); NAKAMURA, Kentaro, Kyoto-shi, Kyoto 601-8510 (JP)
(74) Representative: Müller Hoffmann & Partner
(86) International application number: PCT/JP2024/043601
(87) International publication number: WO 2025/142454

(57) **Abstract**

A gas analysis device that analyzes a target gas containing hydrogen gas as a major component and one or more impurity gases as one or more minor components includes: a comparison chamber into which hydrogen gas is introduced, a first resistance element through which a constant current flows and which generates heat being disposed in the comparison chamber; a measurement chamber into which the target gas is introduced, a second resistance element through which a constant current flows and which generates heat being disposed in the measurement chamber; a bridge circuit formed by including the first resistance element and the second resistance element in a closed circuit, and detects the electrical resistivity of the second resistance element or a related value of the electrical resistivity; a calibration curve data storage unit that stores calibration curve data indicating a relationship between the electrical resistivity of the second resistance element or the related value of the electrical resistivity detected by the bridge circuit and the concentration of a span gas, using hydrogen gas as a base gas and a predetermined single-component gas other than hydrogen gas as the span gas; and a concentration calculation unit that estimates, based on the electrical resistivity of the second resistance element or the related value of the electrical resistivity detected by the bridge circuit and the calibration curve data, the total concentration of the impurity gases in the target gas in terms of a gas component having a thermal conductivity equal to or higher than the thermal conductivity of oxygen.

## Description

### Technical Field

The present invention relates to a thermal-conductivity-based gas analysis device that measures the concentration of a predetermined gas component by using a difference in thermal conductivity between two components, a gas analysis method, and a program for the gas analysis device.

### Background Art

A fuel cell uses the principle of electrolysis of water, and generates electricity by causing hydrogen and oxygen to react chemically. As the hydrogen gas, which is the fuel supplied to the fuel cell, a high-purity hydrogen gas of, for example, 99.98 vol% or higher (which means that the total concentration of impurity gases is 200 ppm or lower) is required.

Therefore, in a conventional hydrogen gas production process, to ensure a high purity of hydrogen gas at a certain level or higher, a portion of produced hydrogen gas is measured by gas chromatography to check the purity. By this method, however, only batch measurement in which the measurement target is stored in a container and is measured can be performed, and the purity of produced hydrogen gas cannot be measured continuously. It is also conceivable to measure the purity of hydrogen gas by measuring the concentrations of the impurity gases contained together with the hydrogen gas. However, there is a problem in that a measuring instrument needs to be prepared for each type of impurity gas, such as a methane meter or a CO₂ meter, which leads to higher costs.

### Citation List

### Patent Literature

Patent Literature 1: JP 2016-042054 A

### Summary of Invention

### Technical Problem

The present invention has been made to solve the problem described above, and a main object of the present invention is to continuously monitor the concentration of produced hydrogen gas with a low-cost configuration, and make it possible to ensure purity of the hydrogen gas.

### Solution to Problem

To solve the above problem, the present inventors focused on a thermal-conductivity-based gas analyzer that measures the concentration of a predetermined gas component, using the difference in thermal conductivity between two components. As disclosed in Patent Literature 1, for example, a thermal-conductivity-based gas analyzer uses the difference in thermal conductivity between a reference gas sealed in a reference chamber and an analysis target gas introduced into a measurement chamber, and can continuously measure, for example, the concentration of the hydrogen gas contained in the analysis target gas. Specifically, in the gas analyzer, a platinum wire (electrical resistance element) through which a constant current flows and which generates heat is disposed in each of the reference chamber and the measurement chamber, and each platinum wire is combined with two external fixed resistors to form a bridge circuit. When the concentration of a measurement target component in the analysis target gas changes, the thermal conductivity in the measurement chamber changes, and as a result, the temperature of the platinum wire changes. The bridge circuit takes out the temperature change as a change in electrical resistance value, and the change in electrical resistance value is converted into the concentration of the measurement target component.

However, the thermal-conductivity-based gas analyzer has a drawback in that, in an environment in which a large number of coexisting components are present, the thermal-conductivity-based gas analyzer is affected by interference, and an error occurs in a measured value. In a hydrogen gas production process, a large number of impurity components such as methane gas and propane gas are produced secondarily in addition to hydrogen gas, and therefore, a thermal-conductivity-based gas analyzer has been considered unsuitable for monitoring hydrogen gas concentration.

The present inventors have conducted intensive studies, and conceived using a thermal-conductivity-based gas analyzer not to directly measure the concentration of hydrogen gas, but to measure the total concentration of a plurality of types of impurity gases contained in the analysis target gas, and calculate the concentration of hydrogen gas, using the measured total concentration of impurity gases. Here, in an environment in which a large number of coexisting components are present, the concentration of each impurity gas cannot be measured accurately as described above. However, if the plurality of types of coexisting impurity gases is regarded as a predetermined single gas component and, moreover, is regarded as a single gas component having a thermal conductivity equal to or higher than that of oxygen gas, which has a comparatively high thermal conductivity among impurity gases generated in the hydrogen production process, and the concentration of the single gas component is then calculated, the total concentration of the plurality of types of impurity gases present can be estimated to be higher than the actual total concentration, though cannot be calculated accurately, and as a result, the concentration of hydrogen gas derived from the result can also be estimated to be lower than the actual concentration. Thus, the present inventors have found that a decrease in the purity of hydrogen gas can be monitored on the safe side, and arrived at the present invention.

That is, a gas analysis device according to the present invention analyzes a target gas containing hydrogen gas as a major component and one or more impurity gases as one or more minor components, and includes: a comparison chamber into which hydrogen gas is introduced, a first resistance element through which a constant current flows and which generates heat being disposed in the comparison chamber; a measurement chamber into which the target gas is introduced, a second resistance element through which a constant current flows and which generates heat being disposed in the measurement chamber; a bridge circuit that is formed by including the first resistance element and the second resistance element in a closed circuit, and detects an electrical resistivity of the second resistance element or a related value of the electrical resistivity; a calibration curve data storage unit that stores calibration curve data indicating a relationship between the electrical resistivity of the second resistance element or the related value of the electrical resistivity detected by the bridge circuit and a concentration of a span gas, using hydrogen gas as a base gas and a predetermined single-component gas other than hydrogen gas as the span gas; and a concentration calculation unit that estimates, based on the electrical resistivity of the second resistance element or the related value of the electrical resistivity detected by the bridge circuit and the calibration curve data, a total concentration of the impurity gases in the target gas in terms of a gas component having a thermal conductivity equal to or higher than a thermal conductivity of oxygen.

In such a configuration, one or more impurity gases contained in the target gas are regarded as a single gas component having a thermal conductivity equal to or higher than the thermal conductivity of oxygen, and the concentration of the single gas component is calculated. Thus, the total concentration of the impurity gases contained in the target gas can be estimated to be higher than the actual total concentration. As the concentration of the impurity gases estimated in this manner is used, the concentration of the hydrogen gas can also be calculated by estimating the concentration of the hydrogen gas to be lower than the actual concentration, and thus, decreases in the purity of the hydrogen gas can be monitored on the safe side.

Furthermore, there is no need to provide an analyzer for each type of impurity gas, and the concentration can be measured while the target gas is introduced into the measurement chamber. Thus, the concentration of produced hydrogen gas can be monitored continuously with a low-cost configuration.

Note that, in the present specification, a major component means a gas component that occupies "85 vol% or more of the target gas".

As a mode that markedly produces the effects of the gas analysis device, there is a mode in which the target gas contains a plurality of types of impurity gases.

In the gas analysis device, the concentration calculation unit preferably estimates the total concentration of the impurity gases in the target gas, in terms of the gas component having the highest thermal conductivity among the impurity gases contained in the target gas.

In this manner, the one or more impurity gases contained in the target gas are regarded as a single gas component having the highest thermal conductivity among the impurity gases contained in the target gas (that is, a gas component having a small difference in thermal conductivity from hydrogen gas, and low sensitivity), and the concentration of the single gas component is then calculated. Thus, the total concentration of the impurity gases contained in the target gas can be estimated more reliably to be higher than the actual total concentration. As the concentration of the impurity gases estimated in this manner is used, the concentration of the hydrogen gas can also be calculated by estimating the concentration of the hydrogen gas more reliably to be lower than the actual concentration, and decreases in the purity of the hydrogen gas can be reliably monitored on the safe side.

Note that the impurity gases contained in the target gas mean gases that are unavoidably and secondarily produced or mixed in during a process of producing hydrogen gas, which is the major component, or gases (for example, air) that are mixed in during the production process, and exclude gases that are artificially mixed in and are unrelated to the hydrogen generation reaction. The same applies below.

Further, in the gas analysis device, the predetermined single-component gas is preferably the gas having the highest thermal conductivity among the impurity gases contained in the target gas.

In this manner, the total concentration of the impurity gases in the target gas can be calculated directly (that is, without interposition of an intermediate calculation) in terms of the gas component having the highest thermal conductivity among the impurity gases contained in the target gas.

Specific modes of the predetermined single-component gas include one selected from the group consisting of methane gas, carbon monoxide gas, carbon dioxide gas, nitrogen gas, and oxygen gas.

In the gas analysis device, the predetermined single-component gas is preferably methane gas.

In the hydrogen gas production process, components such as methane, propane, water vapor, carbon monoxide, carbon dioxide, nitrogen, and oxygen may be produced as impurity gases, and, if a calibration curve is prepared using methane, which has the highest thermal conductivity among these components, as the span gas, a decrease in the purity of hydrogen gas can be detected more reliably.

Specific modes of the gas analysis device include a mode in which the concentration calculation unit calculates the concentration of the hydrogen gas in the target gas, using the estimated total concentration of the impurity gases.

Specific modes of the gas analysis device include a mode in which the bridge circuit includes: the closed circuit obtained by combining the first resistance element and the second resistance element, which have the same specifications as each other, and a third resistance element and a fourth resistance element, which are disposed outside the measurement chamber and the comparison chamber and have the same specifications as each other; a voltage input circuit that inputs a bridge voltage to the closed circuit; and an output voltage measurement circuit that measures an output voltage from the closed circuit.

Further, a gas analysis method of the present invention is a method for analyzing a target gas containing hydrogen gas as a major component and one or more impurity gases as one or more minor components, and includes: a step of introducing hydrogen gas into a comparison chamber in which a first resistance element through which a constant current flows and which generates heat is disposed; a step of introducing the target gas into a measurement chamber in which a second resistance element through which a constant current flows and which generates heat is disposed; a step of detecting an electrical resistivity of the second resistance element or a related value of the electrical resistivity with a bridge circuit formed by including the first resistance element and the second resistance element in a closed circuit; a step of storing calibration curve data indicating a relationship between the electrical resistivity of the second resistance element or the related value of the electrical resistivity detected with the bridge circuit and a concentration of a span gas, using hydrogen gas as a base gas and a predetermined single-component gas other than hydrogen gas as the span gas; and a step of estimating, based on the electrical resistivity of the second resistance element or the related value of the electrical resistivity detected with the bridge circuit and the calibration curve data, a total concentration of the impurity gases in the target gas in terms of a gas component having a thermal conductivity equal to or higher than a thermal conductivity of oxygen.

Such a gas analysis method can produce the same effects as those of the gas analysis device of the present invention described above.

Further, a program for a gas analysis device of the present invention that analyzes a target gas containing hydrogen gas as a major component and one or more impurity gases as one or more minor components, and includes: a comparison chamber into which hydrogen gas is introduced, a first resistance element through which a constant current flows and which generates heat being disposed in the comparison chamber; a measurement chamber into which the target gas is introduced, a second resistance element through which a constant current flows and which generates heat being disposed in the measurement chamber; and a bridge circuit that is formed by including the first resistance element and the second resistance element in a closed circuit, and detects an electrical resistivity of the second resistance element or a related value of the electrical resistivity, the program causing a computer to exhibit functions as: a calibration curve data storage unit that stores calibration curve data indicating a relationship between the electrical resistivity of the second resistance element or the related value of the electrical resistivity detected by the bridge circuit and a concentration of a span gas, using hydrogen gas as a base gas and a predetermined single-component gas other than hydrogen gas as the span gas; and as a concentration calculation unit that estimates, based on the electrical resistivity of the second resistance element or the related value of the electrical resistivity detected by the bridge circuit and the calibration curve data, a total concentration of the impurity gases in the target gas in terms of a gas component having a thermal conductivity equal to or higher than a thermal conductivity of oxygen.

Such a program for a gas analysis device can produce the same effects as those of the gas analysis device of the present invention described above.

### Advantageous Effects of Invention

According to the present invention described above, the concentration of produced hydrogen gas can be monitored continuously with a low-cost configuration, and purity of the generated hydrogen gas can be ensured.

### Brief Description of Drawings

FIG. 1 is a diagram schematically showing a configuration of a gas analysis device according to one embodiment of the present invention.
FIG. 2 is a diagram schematically showing a configuration of a bridge circuit of the embodiment.
FIG. 3 is a functional block diagram of the gas analysis device of the embodiment.

### Description of Embodiments

In the following, a gas analysis device 100 according to one embodiment of the present invention will be described with reference to the drawings.

The gas analysis device 100 of the present embodiment continuously monitors the purity of generated hydrogen gas in, for example, a process of producing hydrogen gas for fuel cells. The gas analysis device 100 is of a so-called thermal conductivity type that measures the concentration of a predetermined gas species by using the difference in thermal conductivity between two different gas components, and is designed to analyze a mixed gas containing hydrogen gas as a major component and a plurality of types of impurity gases as minor components, generated in a hydrogen generation process, (the mixed gas will be hereinafter referred to as the target gas), and to measure the concentration (purity) of the hydrogen gas in the target gas. Note that the hydrogen gas, which is the major component forming the target gas, is derived from natural gas, and the target gas contains, as impurity gases, at least one of methane gas and propane gas.

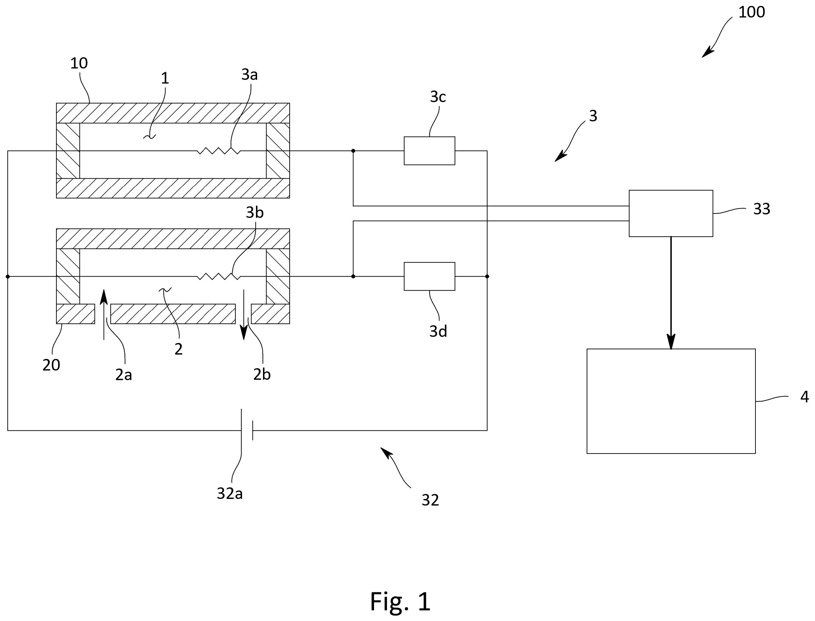

Specifically, as shown in FIG. 1, the gas analysis device 100 includes a comparison chamber 1 into which a reference gas is introduced, a measurement chamber 2 into which the target gas is introduced, a bridge circuit 3 that detects a change in thermal conductivity of the target gas introduced into the measurement chamber 2, and an arithmetic device 4.

The comparison chamber 1 is a chamber into which the reference gas, which is a single-component gas having an invariable concentration, is introduced. The comparison chamber 1 is formed with, for example, a comparison cell 10 formed of a metal material, and the reference gas is sealed in the comparison chamber 1. The metal cell forming the comparison chamber 1 is adjusted to a constant temperature by a temperature control mechanism not shown in the drawing.

The measurement chamber 2 is formed with, for example, a measurement cell 20 formed of a metal material. The measurement chamber 2 has a gas introduction port 2a through which the target gas that is externally supplied is introduced, and a gas discharge port 2b through which the target gas is discharged outside. The target gas is continuously introduced into the measurement chamber 2 through the gas introduction port 2a, and at the same time, the target gas is continuously discharged outside from the measurement chamber 2 through the gas discharge port 2b. The metal cell forming the measurement chamber 2 is adjusted to a constant temperature by a temperature control mechanism not shown in the drawing.

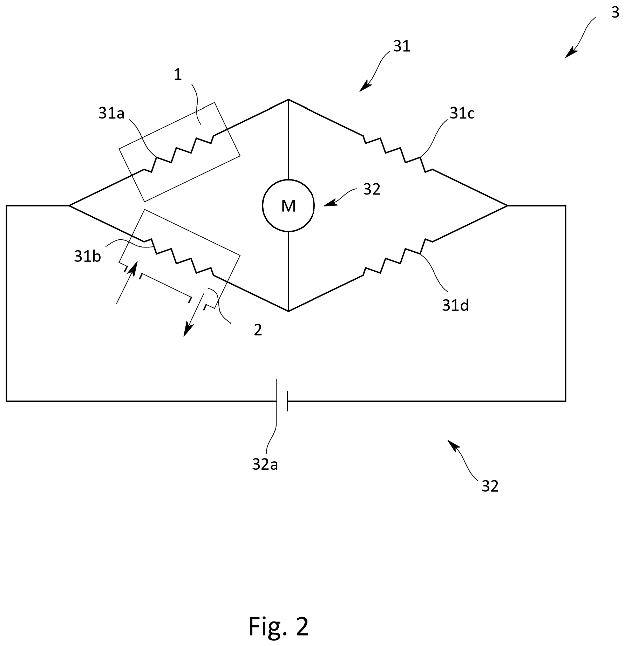

As shown in FIG. 2, the bridge circuit 3 includes a closed circuit 31 formed by combining four electrical resistance elements, a voltage input circuit 32 that inputs a voltage (bridge voltage) to the closed circuit 31, and an output voltage measurement circuit that measures a voltage output from the closed circuit 31. The voltage input circuit 32 includes a constant-current power supply 32a that inputs a constant current to the bridge circuit 3. The term "constant current" means not only a current whose value indicates a constant value without fluctuation, but also a current whose value fluctuates within a range of ±10 mA or less.

The closed circuit 31 is a closed circuit 31 formed by combining a first resistance element 3a and a second resistance element 3b, which have the same specifications as each other, and a third resistance element 3c and a fourth resistance element 3d, which have the same specifications as each other. In the bridge circuit 3, the first resistance element 3a and the third resistance element 3c are connected so as to be in a diagonal relationship with each other, and the second resistance element 3b and the fourth resistance element 3d are connected so as to be in a diagonal relationship with each other.

The first resistance element 3a is formed with a metal wire such as a platinum wire, and is set in the comparison chamber 1 in a state of being insulated from the metal comparison cell 10. The first resistance element 3a generates heat in the comparison chamber 1 when the constant current output from the constant-current power supply 32a flows through the first resistance element 3a. Since the reference gas, which is a single-component gas having an invariable concentration, is sealed in the comparison chamber 1 as described above, the thermal conductivity in the comparison chamber 1 has a constant value. Thus, the temperature and the electrical resistivity of the first resistance element 3a are maintained at substantially constant values.

The second resistance element 3b is also formed with a metal wire such as a platinum wire, and is set in the measurement chamber 2 in a state of being insulated from the metal measurement cell 20. The second resistance element 3b generates heat in the measurement chamber 2 when the constant current output from the constant-current power supply 32a flows through the second resistance element 3b. As described above, the target gas is continuously introduced into the measurement chamber 2. However, when the concentration of a component contained in the target gas changes, the thermal conductivity in the measurement chamber 2 changes, leading to a change in the temperature of the second resistance element 3b and a change in the electrical resistivity of the second resistance element 3b.

The third resistance element 3c and the fourth resistance element 3d of the present embodiment are both fixed manganin resistors, are disposed outside the measurement chamber 2 and the comparison chamber 1, and are maintained at substantially constant temperatures and electrical resistivities.

The bridge circuit 3 is designed to measure the electrical resistivity of the second resistance element 3b, which changes as the target gas flows in the measurement chamber 2, and to output the value of the electrical resistivity. Specifically, when the electrical resistivity of the second resistance element 3b changes due to the target gas flowing in the measurement chamber 2, the output voltage measured by an output voltage measurement circuit 33 changes. The output voltage measurement circuit 33 converts the change in the measured output voltage into the electrical resistivity of the second resistance element 3b by a predetermined algorithm, and outputs the value of the electrical resistivity.

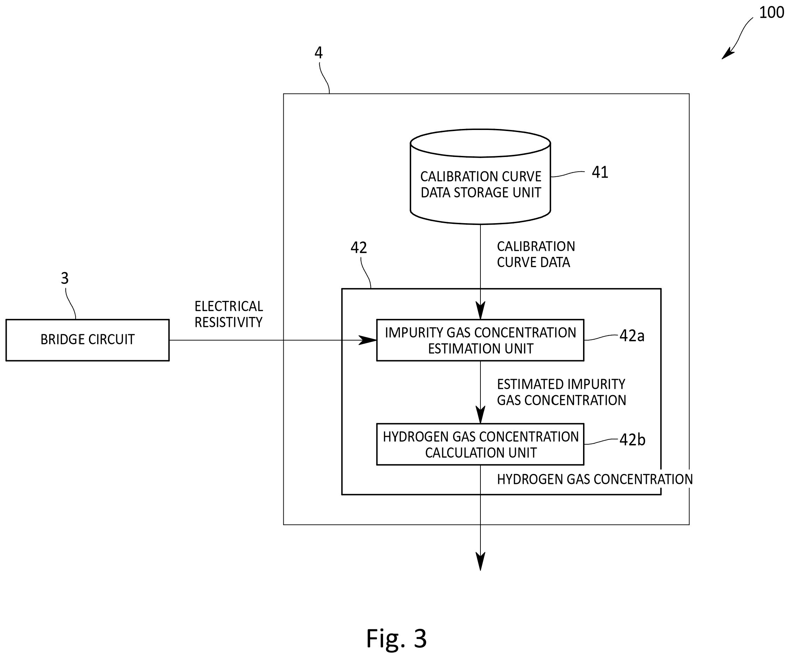

The arithmetic device 4 is a computer that includes a CPU, a memory, an A/D converter, a D/A converter, various input and output devices, and the like, and, by the CPU and peripheral devices cooperating based on a predetermined program stored in the memory, the arithmetic device 4 exhibits at least the functions of a calibration curve data storage unit 41 and a concentration calculation unit 42, as shown in FIG. 3.

The calibration curve data storage unit 41 is set in a predetermined area of the memory, and stores calibration curve data that is prepared in advance using hydrogen gas as a base gas and a predetermined single-component gas other than hydrogen gas as a span gas, and indicates a relationship between the electrical resistivity of the second resistance element 3b detected by the bridge circuit 3 and the concentration of the span gas.

In the present embodiment, the calibration curve data is prepared using, as the span gas, a single-component gas having a thermal conductivity equal to or higher than that of oxygen. More specifically, in the present embodiment, the calibration curve data is prepared using, as the span gas, one gas having the highest thermal conductivity among the plurality of types of impurity gases contained in the target gas, and still more specifically, the calibration curve data is prepared using methane gas as the span gas.

The concentration calculation unit 42 includes an impurity gas concentration estimation unit 42a that estimates and calculates the total concentration of the impurity gases in the target gas, and a hydrogen gas concentration calculation unit 42b that calculates the hydrogen gas concentration in the target gas, based on the estimated impurity gas concentration (estimated impurity gas concentration).

The impurity gas concentration estimation unit 42a estimates and calculates the total concentration of the impurity gases in the target gas, on the assumption that the impurity gases contained in the target gas have thermal conductivities equal to or higher than that of oxygen. The impurity gas concentration estimation unit 42a of the present embodiment estimates and calculates the total concentration of the impurity gases in the target gas, on the assumption that, among the plurality of types of impurity gases contained in the target gas, only one gas having the highest thermal conductivity, or more specifically only methane gas, is contained in the target gas as the impurity gas. Specifically, the impurity gas concentration estimation unit 42a estimates and calculates the total concentration of the impurity gases in the target gas, based on the electrical resistivity of the second resistance element 3b detected by the bridge circuit 3 and the calibration curve data stored in the calibration curve data storage unit 41. As described above, a change in the electrical resistivity of the second resistance element 3b detected by the bridge circuit 3 is caused by a change in thermal conductivity due to a change in concentration of the plurality of types of impurity gases contained in the target gas. On the other hand, the calibration curve data is prepared using methane gas, which is a single-component gas, as the span gas. That is, the impurity gas concentration estimation unit 42a calculates the total concentration of the impurity gases in the target gas, on the assumption that the gases contained in the target gas are only two components, which are hydrogen gas and methane gas, or, in other words, on the assumption that the impurity gas contained in the target gas is of only one type, which is methane gas. It can also be said that the impurity gas concentration estimation unit 42a of the present embodiment calculates the total concentration of the impurity gases in the target gas in terms of methane gas.

The hydrogen gas concentration calculation unit 42b calculates the concentration of the hydrogen gas on the assumption that the remaining portion of the calculated impurity gas concentration is regarded as the hydrogen gas concentration, and outputs the concentration of the hydrogen gas to a display or the like.

With the gas analysis device 100 of the present embodiment designed as above, one or more impurity gases included in the target gas are regarded as the single gas component having the highest thermal conductivity among the impurity gases included in the target gas (that is, a gas component having a small difference in thermal conductivity from the hydrogen gas, and low sensitivity), and the concentration of the single gas component is calculated, so that the total concentration of the impurity gases contained in the target gas can be estimated to be higher than the actual total concentration. By using the concentration of the impurity gases estimated in this manner, the concentration of the hydrogen gas can also be calculated by estimating the concentration of the hydrogen gas to be lower than the actual concentration, and a decrease in the purity of the hydrogen gas can be reliably monitored on the safe side. Furthermore, there is no need to provide an analyzer for each type of impurity gas, and the concentration can be measured while the target gas is introduced into the measurement chamber 2. Thus, the concentration of produced hydrogen gas can be monitored continuously with a low-cost configuration.

Note that the present invention is not limited to the embodiment described above.

For example, in the embodiment described above, the target gas contains a plurality of types of impurity gases. However, the target gas is not limited to this. In another embodiment, the target gas may contain hydrogen gas as a major component, and contain only one type of impurity gas as a minor component.

In the gas analysis device 100 of the embodiment described above, the impurity gas concentration estimation unit 42a calculates the total concentration of the impurity gases in the target gas by using calibration curve data prepared using, as the span gas, one gas having the highest thermal conductivity among the plurality of types of impurity gases contained in the target gas. However, the gas analysis device 100 is not limited to this. In the gas analysis device 100 of another embodiment, the impurity gas concentration estimation unit 42a may calculate the total concentration of the impurity gases in the target gas by using calibration curve data prepared using, as the span gas, a gas other than "one gas having the highest thermal conductivity among the plurality of types of impurity gases contained in the target gas" (for example, oxygen gas, nitrogen gas, or the like; the gas may be a gas not contained in the target gas). In this case, the impurity gas concentration estimation unit 42a may calculate the total concentration of the impurity gases in the target gas in terms of the gas having the highest thermal conductivity by multiplying a predetermined coefficient stored in advance by the span gas concentration calculated based on the calibration curve data and the electrical resistivity of the second resistance element 3b detected by the bridge circuit 3.

Further, the gas analysis device 100 of the embodiment described above includes the hydrogen gas concentration calculation unit 42b and outputs the calculated hydrogen gas concentration. However, the gas analysis device 100 is not limited to this. In another embodiment, the gas analysis device 100 may exclude the hydrogen gas concentration calculation unit 42b, and may be designed to output only the impurity gas concentration calculated by estimation on a display or the like.

Further, the concentration calculation unit 42 of the embodiment described above calculates the total concentration of the impurity gases and the hydrogen gas concentration, using calibration curve data prepared using hydrogen gas as the base gas and methane gas as the span gas. However, the concentration calculation unit 42 is not limited to this. The span gas used for preparation of the calibration curve data may be changed as appropriate, depending on the origin of the hydrogen gas forming the target gas. For example, in a case where the hydrogen gas forming the target gas is derived from electrolysis of water, the target gas contains, as an impurity gas, at least one of water vapor, oxygen gas, and nitrogen gas. In this case, the total concentration of the impurity gases in the target gas may be calculated by using calibration curve data prepared using hydrogen gas as the base gas and nitrogen gas having the highest thermal conductivity as the span gas.

Further, in the embodiment described above, the calibration curve data indicates a relationship between the electrical resistivity of the second resistance element 3b detected by the bridge circuit 3 and the concentration of the span gas. However, the calibration curve data is not limited to this. In another embodiment, the calibration curve data may indicate a relationship between a related value of the electrical resistivity of the second resistance element 3b detected by the bridge circuit 3 (for example, an output voltage or an output current detected by the output voltage measurement circuit 33 of the bridge circuit 3) and the concentration of the span gas.

The gas analysis device 100 of another embodiment may be designed to output the calculated impurity gas concentration to a display or the like, instead of the calculated hydrogen gas concentration or together with the calculated hydrogen gas concentration.

Further, in the gas analysis device 100 of the embodiment described above, the impurity gas concentration estimation unit 42a calculates the total concentration of the impurity gases in the target gas in terms of methane gas. However, the impurity gas concentration estimation unit 42a is not limited to this. The impurity gas concentration estimation unit 42a of another embodiment may be designed to estimate the total concentration of the impurity gases in the target gas in terms of a gas component having a thermal conductivity equal to or higher than that of oxygen (for example, in terms of oxygen gas, in terms of carbon monoxide gas, in terms of nitrogen gas, in terms of air gas, or in terms of ammonia gas).

Note that, in another embodiment, the target gas to be analyzed by the gas analysis device 100 may be a mixed gas not containing hydrogen gas as a major component.

Other than the above, various modifications and combinations of embodiments may be made, as long as the modifications and combinations do not depart from the gist of the present invention.

### Industrial Applicability

According to the present invention described above, the concentration of produced hydrogen gas can be monitored continuously with a low-cost configuration, and the purity of the produced hydrogen gas can be ensured.

### Reference Signs List

- 100: gas analysis device
- 1: comparison chamber
- 2: measurement chamber
- 3: bridge circuit
- 3a: first resistance element
- 3b: second resistance element
- 41: calibration curve data storage unit
- 42: concentration calculation unit

## Claims

1. A gas analysis device that analyzes a target gas containing hydrogen gas as a major component and one or more impurity gases as one or more minor components, the gas analysis device comprising:
a comparison chamber into which hydrogen gas is introduced, a first resistance element through which a constant current flows and which generates heat being disposed in the comparison chamber;
a measurement chamber into which the target gas is introduced, a second resistance element through which a constant current flows and which generates heat being disposed in the measurement chamber;
a bridge circuit that is formed by including the first resistance element and the second resistance element in a closed circuit, and detects an electrical resistivity of the second resistance element or a related value of the electrical resistivity;
a calibration curve data storage unit that stores calibration curve data indicating a relationship between the electrical resistivity of the second resistance element or the related value of the electrical resistivity detected by the bridge circuit and a concentration of a span gas, using hydrogen gas as a base gas and a predetermined single-component gas other than hydrogen gas as the span gas; and
a concentration calculation unit that estimates, based on the electrical resistivity of the second resistance element or the related value of the electrical resistivity detected by the bridge circuit and the calibration curve data, a total concentration of the one or more impurity gases in the target gas in terms of a gas component having a thermal conductivity equal to or higher than a thermal conductivity of oxygen.

2. The gas analysis device according to claim 1, wherein the target gas contains a plurality of types of impurity gases.

3. The gas analysis device according to claim 1 or 2, wherein the concentration calculation unit estimates the total concentration of the impurity gases in the target gas in terms of a gas component having a highest thermal conductivity among the impurity gases contained in the target gas.

4. The gas analysis device according to any one of claims 1 to 3, wherein the predetermined single-component gas is a gas having a highest thermal conductivity among the impurity gases contained in the target gas.

5. The gas analysis device according to any one of claims 1 to 4, wherein the predetermined single-component gas is one gas selected from the group consisting of methane gas, carbon monoxide gas, carbon dioxide gas, nitrogen gas, and oxygen gas.

6. The gas analysis device according to claim 5, wherein the predetermined single-component gas is methane gas.

7. The gas analysis device according to any one of claims 1 to 6, wherein the concentration calculation unit calculates the concentration of the hydrogen gas in the target gas, using the estimated total concentration of the impurity gases.

8. The gas analysis device according to any one of claims 1 to 7, wherein the bridge circuit includes
the closed circuit obtained by combining the first resistance element and the second resistance element, which have same specifications as each other, and a third resistance element and a fourth resistance element that are disposed outside the measurement chamber and the comparison chamber, and have same specifications as each other;
a voltage input circuit that inputs a bridge voltage to the closed circuit; and
an output voltage measurement circuit that measures an output voltage from the closed circuit.

9. A gas analysis method for analyzing a target gas containing hydrogen gas as a major component and one or more impurity gases as one or more minor components, the gas analysis method comprising:
a step of introducing hydrogen gas into a comparison chamber in which a first resistance element through which a constant current flows and which generates heat is disposed;
a step of introducing the target gas into a measurement chamber in which a second resistance element through which a constant current flows and which generates heat is disposed;
a step of detecting an electrical resistivity of the second resistance element or a related value of the electrical resistivity with a bridge circuit formed by including the first resistance element and the second resistance element in a closed circuit;
a step of storing calibration curve data indicating a relationship between the electrical resistivity of the second resistance element or the related value of the electrical resistivity detected with the bridge circuit and a concentration of a span gas, using hydrogen gas as a base gas and a predetermined single-component gas other than hydrogen gas as the span gas; and
a step of estimating, based on the electrical resistivity of the second resistance element or the related value of the electrical resistivity detected with the bridge circuit and the calibration curve data, a total concentration of the one or more impurity gases in the target gas in terms of a gas component having a thermal conductivity equal to or higher than a thermal conductivity of oxygen.

10. A program for a gas analysis device that analyzes a target gas containing hydrogen gas as a major component and one or more impurity gases as one or more minor components, the gas analysis device including: a comparison chamber into which hydrogen gas is introduced, a first resistance element through which a constant current flows and which generates heat being disposed in the comparison chamber; a measurement chamber into which the target gas is introduced, a second resistance element through which a constant current flows and which generates heat being disposed in the measurement chamber; and a bridge circuit that is formed by including the first resistance element and the second resistance element in a closed circuit, and detects an electrical resistivity of the second resistance element or a related value of the electrical resistivity, the program causing a computer to exhibit functions as:
a calibration curve data storage unit that stores calibration curve data indicating a relationship between the electrical resistivity of the second resistance element or the related value of the electrical resistivity detected by the bridge circuit and a concentration of a span gas, using hydrogen gas as a base gas and a predetermined single-component gas other than hydrogen gas as the span gas; and
a concentration calculation unit that estimates, based on the electrical resistivity of the second resistance element or the related value of the electrical resistivity detected by the bridge circuit and the calibration curve data, a total concentration of the one or more impurity gases in the target gas in terms of a gas component having a thermal conductivity equal to or higher than a thermal conductivity of oxygen.
